(19) Europäisches Patentamt
European Patent Office
Office européen des brevets

(11) Veröffentlichungsnummer: **0 130 537**
**B1**

(12) # EUROPÄISCHE PATENTSCHRIFT

(45) Veröffentlichungstag der Patentschrift: **25.01.89**

(51) Int. Cl.⁴: **G 01 N 33/92,** G 01 N 33/96

(21) Anmeldenummer: **84107339.8**

(22) Anmeldetag: **26.06.84**

(54) **Verfahren und Mittel zur raschen und vollständigen Beseitigung einer Trübung in einer biologischen Flüssigkeit.**

(30) Priorität: **02.07.83 DE 3323949**

(43) Veröffentlichungstag der Anmeldung: **09.01.85 Patentblatt 85/2**

(45) Bekanntmachung des Hinweises auf die Patenterteilung: **25.01.89 Patentblatt 89/4**

(84) Benannte Vertragsstaaten: **AT BE CH DE FR GB IT LI LU NL SE**

(56) Entgegenhaltungen:
**EP-A-0 004 857**
**EP-A-0 008 338**
**EP-A-0 041 704**
**EP-A-0 076 211**
**FR-A-2 393 291**

(73) Patentinhaber: **BOEHRINGER MANNHEIM GMBH, Sandhofer Strasse 116, D-6800 Mannheim 31 (DE)**

(72) Erfinder: **Siedel, Joachim, Dr. rer. nat., Bahnhofstrasse 6, D-8131 Bernried (DE)**
Erfinder: **Staepels, Johnny, Jahnstrasse 20, D-8130 Starnberg (DE)**
Erfinder: **Ziegenhorn, Joachim, Dr. rer. nat., Ina-Seidel- Weg 1, D-8130 Starnberg (DE)**

**EP 0 130 537 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft ein Verfahren und Mittel zur raschen und vollständigen Beseitigung einer Trübung in einer biologischen Flüssigkeit, insbesondere in menschlichem Blutserum oder -plasma, unter Verwendung von oberflächenaktiven Mitteln.

Trübungen im menschlichen Blutserum werden durch einen überhöhten Gehalt an triglyceridreichen Lipoproteinpartikeln wie Chylomikronen und VLDL ("Very Low Density Lipoproteins") hervorgerufen. Man spricht in diesem Fall von "lipämischem" oder "hyperlipoproteinämischem" Serum. Derartige Trübungen stellen für die Durchführbarkeit photometrischer Analysen von Serumbestandteilen in der klinischchemischen Diagnostik ein erhebliches Problem dar. Dies gilt insbesondere dann, wenn die Konzentration des zu bestimmenden Bestandteils im Serum sehr gering ist, wie z. B. die von Spurenelementen, und für eine ausreichende Meßgenauigkeit dem Analysenreagens verhältnismäßig große Serummengen zugesetzt werden müssen (Volumenverhältnis Serum : Analysenreagens ⩾ 0,15). Hier kann auch schon bei geringem Lipämiegrad die durch das Serum im Reagens verursachte Trübung zur Überschreitung des Linearitätsbereichs des Photometers führen und damit die Messung erheblich beeinträchtigen oder unmöglich machen.

Es hat sich aber auch gezeigt, daß verschiedene immunologische Testverfahren, insbesondere solche, bei denen die durch Immunpräzipitationsreaktionen entstehenden Trübungen nephelometrisch oder turbidimetrisch gemessen werden, und bei denen vergleichsweise geringe Serummengen im Testansatz erforderlich sind, durch Eigentrübungen des Probenmaterials empfindlich gestört werden können. Als Beispiel sei hier erwähnt die immuno-nephelometrische Bestimmung von Serum-Apolipoproteinen (Clin. Chem. 28, (1982) 1153-1158; Clin. Chem. 29, (1983) 120-125) sowie die radioimmunologische Bestimmung von β-Choriogonadotropin und Prostata-Saure-Phosphatase (Clin.Chem. 28, (1982) 2325).

Die vollständige Beseitigung von Trübungen ("Aufklarung") in lipämischem Serum ist daher für die klinische Analyse von außerordentlicher Bedeutung.

Aus der Literatur sind schon verschiedene Verfahren und Mittel zur Beseitigung von Trübungen in biologischen Flüssigkeiten bekannt.

So ist z. B. in Clin. Chem. 29, (1983) 120-125 ein Verfahren beschrieben, bei dem Serumtrübungen durch Ausschütteln mit einem Gemisch organischer Lösungsmittel entfernt werden. Diese Methode erfordert einen zusätzlichen Verfahrensschritt. Darüber hinaus kann es insbesondere bei stark lipämischen Seren zu unkontrollierbaren Volumenveränderungen des Probenmaterials kommen und damit zu einer Verfälschung der Meßergebnisse. Schließlich ist bei Einsatz dieses Verfahrens auch nicht mehr die Bestimmung von Serumbestandteilen möglich, die bei der Extraktion ganz oder teilweise entfernt werden.

Das Gleiche gilt für ein Verfahren, bei dem die trübungsverursachenden Lipoproteinpartikel durch Zusatz von Polyanionen wie Phospherwolframsäure/Magnesiumchlorid aus dem Serum ausgefällt und abzentrifugiert werden (Clin. Chem. 28, (1983) 1153-1158).

Aus Gründen der Meßgenauigkeit und der Wirtschaftlichkeit photometrischer Serumanalysen ist es zweckmäßig, die Aufklarung direkt in dem zur Bestimmung des jeweiligen Serumbestandteils eingesetzten Analysenreagens durchzuführen und zwar vollständig und innerhalb weniger Minuten (⩽ 10 min) sowie üblicherweise in einem Temperaturbereich zwischen 15 und 40°C.

Ein solches Verfahren, das aber zum Teil nur zur Verminderung der Trübung in einer Serum- oder Plasmaprobe führt, ist beispielsweise aus der DE-OS-2 327 894 bekannt. Hierbei wird dem Analysenreagens eine polyoxyethylierte Laurinsäureverbindung in höheren Konzentrationen hinzugefügt.

In DE-AS-2 724 757 ist ein Mittel zur Beseitigung von Trübungen im Serum beschrieben, das aus einer wäßrigen, gepufferten Lösung von Fettsäurepolyethylen-Glykolestern sowie kurzkettigen aliphatischen Alkoholen, Glykolen oder Polyethylenglykolen bzw. einem Fettalkohol-Polyglykolether besteht. Dieses Mittel ist geeignet, trube Meßlösungen bei einem Volumenverhältnis Serum: Reagens ⩽ 0,1 innerhalb weniger Minuten aufzuklaren. Beispielsweise gelingt bei einer Lösung mit einem Volumenverhältnis von Serum : Reagens = 0,1 bei 20 bis 25°C eine vollständige Aufklarung innerhalb von 5 min nach Durchmischung. Bei einem Volumenverhältnis von Serum : Reagens > 0,15 wird jedoch eine erheblich längere Zeit zur Aufklarung der Meßlösung benötigt. Zur Beseitigung der Trübung in einer Lösung mit einem Volumenverhältnis von Serum: Reagens = 0,16 sind bereits 30 min erforderlich.

Aus Z. Klin. Chem. Klin. Biochem. 3, (1965) 96-99 ist ein Verfahren zur Transferrin-Eisen-Bestimmung im Serum bekannt, wobei dem Analysenreagens ein sekundäres Alkylsulfat ("Teepol 610 S", Shell AG) in hoher Konzentration zugesetzt wird. Beim Vermischen von 0,5 ml Serum und 1,4 ml Reagens wird eine vollständige Trübungsbeseitigung nach 15 min bei Raumtemperatur erreicht.

In der EP-OS-0 041 704 wird schließlich vorgeschlagen, Chylomikronen in wäßrigem Medium mit Hilfe eines Gemisches aus einem Polyethylenglykolether eines Alkanols oder Alkylarylalkohols mit verzweigter Alkankette und einem HLB-Wert von 12 bis 14, einem sekundären Alkylsulfonat mit 10 bis 20 C-Atomen im Molekül sowie gegebenenfalls einem Alkali-p- toluolsulfonat aufzulösen. Die Überprüfung eines in der EP-OS-0 041 704 als besonders geeignet beschriebenen Mittels (Mittel gemäß Beispiel 2) ergab, daß es damit nicht möglich ist, bei einem stark lipämischen Serum und einem Volumenverhältnis von Serum : Reagens ⩾ 0,2 bei 25°C innerhalb von 30 min eine vollständige Aufklarung zu erzielen.

Diese vorbekannten Methoden zur Beseitigung von Trübungen in biologischen Flüssigkeiten weisen somit alle noch z. T. erhebliche Nachteile auf, die im wesentlichen beruhen auf

a) der Notwendigkeit einer Serum-Vorbehandlung in einem zusätzlichen Verfahrensschritt oder, wenn die Aufklarung direkt im Analysenreagens erfolgt,

b) einem begrenzten Aufklarungsvermögen des verwendeten Mittels,
c) einer unzureichenden Aufklarungsgeschwindigkeit (> 10 min), sowie
d) einem eingeschränkten pH- und Temperaturbereich, in dem das verwendete Verfahren oder Mittel wirksam ist, z. B. wenn zur Unterstützung der aufklarenden Wirkung noch lipolytisch aktive Enzyme ("Lipasen") zugesetzt werden.

Ferner findet sich in keiner der aufgeführten Literaturstellen ein Hinweis darauf, daß in dem jeweils verwendeten Aufklarungsreagens auch Antigen-Antikörperreaktionen wie z. B. bei Immunpräzipitationsanalysen von Serum-Apolipoproteinen störungsfrei durchführbar sind.

Um Störungen durch Serumtrübungen bei der immunonephelometrischen Bestimmung eines Serumproteins, insbesondere eines Apolipoproteins, zu vermeiden, wird in der DE-OS-2 829 531 ein Verfahren vorgeschlagen, bei dem die Immunreaktion entweder in Gegenwart eines sehr niedrig konzentrierten kationischcn Tensids ($10^{-3}$ bis $10^{-1}$ Vol-%) oder in Gegenwart eines sehr niedrig konzentrierten nicht-ionischen Tensids ($10^{-3}$ bis $10^{-1}$ Vol-%) und einem lipolytisch aktiven Enzym durchgeführt wird. Da dabei nur sehr geringe Serummengen dem Testansatz hinzugefügt herden müssen, brauchen an das Aufklarungsvermögen des verwendeten Mittels keine hohen Anforderungcn gestellt zu werden.

Bei analogen immunoturbidimetrischen Messungen sind wesentlich höhere Serum-Konzentrationen Im Testansatz erforderlich. Über die Wirksamkeit der Tenside bei diesen erhöhten Serum-Konzentrationen wird in der DE-OS-2 829 531 nichts ausgesagt. Es wird jedoch erwähnt, daß durch Tensid-Konzentrationen über 0,1 Vol-% die immunologische Reaktion zwischen Antikörper und Antigen (im vorliegenden Fall dem Apolipoprotein) gehemmt wird.

Es besteht daher Bedarf an einem Verfahren und Mittel, das auch bei Volumenverhältnissen von Serum : Analysenreagens $\geqslant$ 0,15 eine vollständige und dauerhafte Aufklarung von Serumtrübungen bewirkt. Die Aufklarung soll dabei möglichst rasch, vorzugsweise in weniger als 10 min erfolgen. Die Wirksamkeit soll über einen möglichst großen pH-($3 \leqslant pH \leqslant 9$) und Temperaturbereich ($15 \leqslant T \leqslant 37°C$) gewährleistet sein. Neben der genauen Bestimmung von Spurenelementen soll das Verfahren und Reagens insbesondere auch die störungsfreie Analyse von Proteinen wie Apolipoproteinen mit immunoturbidimetrischen Meßmethoden, selbst bei Verwendung stark lipämischer Seren, ermöglichen. Schließlich sollte das dem Verfahren zugrundeliegende Mittel über einen langen Zeitraum, d. h. mindestens ein Jahr bei Raumtemperatur lagerstabil sein.

Der Erfindung liegt die Aufgabe zugrunde, diesen Bedarf zu befriedigen. Gelöst wird diese Aufgabe durch ein Verfahren zur Beseitigung von Trübungen in biologischen Flüssigkeiten durch Zusatz von oberflächenaktiven Mitteln, dadurch gekennzeichnet, daß als ober flächenaktive Mittel

a) ein polyethoxyliertes Triglycerid mit einem HLB-Wert von 4 bis 14,
b) ein sekundäres n-Alkansulfonat, sowie gegebenenfalls
c) ein weiteres nicht- oder anionisches Tensid

in wäßriger, gegebenenfalls gepufferter Lösung eingesetzt wird.

Als polyethoxyliertes Triglycerid kann z. B. polyethoxyliertes Triolein (I.B. Tagat® TO, Fa. Goldschmidt AG, HLB-Wert 11.3) oder polyethoxyliertes Rizinusöl (z. B. Mulsifan® RT 7 oder Mulsifan® RT 163, Fa. Zschimmer + Schwarz, HLB-Wert ca. 10 bzw. 6) eingesetzt werden. Besonders gute Ergebnisse werden erzielt, wenn man ein poly-ethoxyliertes Triglycerid verwendet, das durch Umsetzung von Ethylenoxid und Rizinusöl im Autoklaven unter alkalischer Katalyse gewonnen wird. Das auf diese Weise hergestellte Produkt enthält durchschnittlich 10 Oxyethylen-Einheiten pro Molekül und ist unter der Bezeichnung Mulsifan® RT 163 im Handel erhältlich.

Der HLB-Wert (hydrophilic-lipophilic-balance) der erfindungsgemäß einsetzbaren polyethoxylierten Triglyceride kann nach bekannten Verfahren bcstimmt werden, vgl. z. B. Stache "Tensid-Taschenbuch", Carl-Hanser-Verlag, München, Wien 1979, Seiten 70-72.

Die Konzentration der polyethoxylierten Triglyceride in der wäßrigen, gepufferten Lösung beträgt 0,5 bis 15, bevorzugt 1,0 bis 12, insbesonderc 2 bis 9 Gew.-%.

Als sekundäre n-Alkansulfonate werden vorzugsweise Verbindungen mit 12 bis 19 C-Atomen im Molekül als Reinsubstanzen oder auch Gemische verschiedener n-Alkansulfonate mit Überwiegend 12 bis 19 C-Atomen im Molekül eingesetzt. Die sekundären n-Alkansulfonate werden bevorzugt als Natriumsalze verwendet. Als erfindungsgemäß besonders günstig hat sich ein sekundäres n-Alkansulfonat (Na-Salz) erwiesen, das durch Sulfoxidation von n-Paraffinen hergestellt wird und eine C-Kettenverteilung von

$< C_{13}$-n-Paraffin = max. 1 %
$C_{13}$-$C_{15}$-n-Paraffin = ca. 58 %
$C_{16}$-$C_{17}$-n-Paraffin = ca. 39 %
$> C_{17}$-n-Paraffin = max. 3 %

aufweist. Ein solches Produkt ist als wasserhaltige Paste mit einem Wirkstoffanteil von 60 % unter dem Namen Hostapur®AT im Handel (Fa. Hoechst AG).

Das erfindungsgemäße sekundäre n-Alkansulfonat ist in der wäßrigen, gegebenenfalls gepufferten Lösung in einer Konzentration von 0,5 bis 10, bevorzugt 1,0 bis 7, insbesondere 1,2 bis 4,8 Gew.-%, bezogen auf den reinen Wirkstoff, enthalten.

Gemische aus den erfindungsgemäßen polyethoxylierten Triglyceriden und sekundären n-Alkansulfonaten in wäßriger, gegebenenfalls gepufferter Lösung bewirken bereits als solche eine hinreichend schnelle Aufklarung trüber Seren. Zur weiteren Beschleunigung der Aufklarungsgeschwindigkeit hat es sich jedoch als günstig erwiesen, diesen Gemischen ein weiteres nicht- oder anionisches Tensid hinzuzufügen.

Als gegebenenfalls zugesetzte weitere nicht-ionische Tenside kommen in Frage gerad- oder verzweigtkettige Alkylalkohol- oder Alkylarylalkohol-polyglykolether mit niedrigem Oxyethylierungsgrad (durchschnittlich 3 bis 7 Oxyethylen-Einheiten pro Molekül). Bevorzugt wird ein Iso-Decanolpolyglykolether mit durchschnittlich 5 Oxyethylen-Einheiten pro Molekül (Lutensol® ON 50, BASF).

Als anionische Tenside sind geeignet Alkyl-aryl-sulfonate, sekundäre Alkylsulfate oder Gemische von Alkansulfonaten mit 13 bis 15 C-Atomen im Molekül und 40 bis 50 % sekundärem Anteil (Mersolat® H, Bayer AG). Besonders bevorzugt wird Natrium-Dodecylbenzolsulfonat (z. B. als Elfan® WA 50 mit einem Wirkstoffgehalt von 50 % von der Fa. Akzo im Handel vertrieben).

Hierbei haben sich in allen Fällen Konzentrationen in der wäßrigen, gegebenenfalls gepufferten Lösung von 0,2 bis 5, bevorzugt von 0,5 bis 3, insbesondere von 1 bis 2 Gew.-%, bezogen auf den reinen Wirkstoff, als günstig erwiesen.

Das erfindungsgemäße Verfahren läßt sich durchführen, indem man die einzelnen Bestandteile, d. h. Wasser oder gegebenenfalls wäßrige Pufferlösung, oberflächenaktive Mittel und biologische Flüssigkeit nacheinander vermischt. Zweckmäßigerweise wird jedoch ein vorgefertigtes Gemisch der oberflächenaktiven Mittel in einer wäßrigen, gegebenenfalls gepufferten Lösung verwendet, das der biologischen Flüssigkeit in einer Menge zugesetzt wird, die zu einer raschen und vollständigen Beseitigung der Trübung führt. Im allgemeinen reichen zur vollständigen Beseitigung der Trübung in 0,2 ml Serum Mengen von 0,5 bis 2 ml des erfindunsgemäßen Mittels aus.

Der pH-Wert des Gemisches kann in weiten Grenzen gewählt werden. Nach dem erfindungsgemäßen Verfahren können ohne weiteres Trübungen bei pH-Werten von 3 bis 9 beseitigt werden. Zur Einstellung des pH-Wertes sind alle gängigen Puffersubstanzen verwendbar, deren pH-Wert zwischen 2,0 und 10,0 liegt. Besonders bevorzugt werden Succinat-, Acetat-, Phosphat oder Trispuffer eingesetzt. Die Pufferkonzentration beträgt zweckmäßigerweise 5 bis 250 mM, insbesondere 10 bis 170 mM.

Die Aufklarungsgeschwindigkeit läßt sich bis zu einer gewissen Grenze durch eine Erhöhung der Ionenstärke im wäßrigen, gegebenenfalls gepufferten Medium günstig beeinflussen. Es hat sich daher als vorteilhaft erwiesen, bei Abwesenheit von Puffersubstanz oder bei niedrigen Pufferkonzentrationen (5 bis 20 mM) der biologischen Flüssigkeit bzw. dem Detergensgemisch ein Salz, beispielsweise Natriumchlorid, oder mehrere Salze zuzusetzen. Die Konzentration an zugesetztem Salz beträgt vorzugsweise 50 bis 200 mM, insbesondere 50 bis 150 mM.

Die Trübungsbeseitigung gelingt nach dem erfindungsgemäßen Verfahren in einem breiten Temperaturintervall ($15° \leqslant T \leqslant 40° C$); bevorzugt wird bei Temperaturen zwischen 20 bis 37° C gearbeitet.

Ein weiterer Gegenstand der Erfindung ist ein Mittel zur raschen und vollständigen Beseitigung einer Trübung in einer biologischen Flüssigkeit, insbesondere menschlichcm Blutserum oder -plasma, dadurch gekennzieichnet, daß es

a) ein polyethoxyliertes Triglycerid mit einem HLB-Wert von 4 bis 14,
b) ein sekundäres n-Alkansulfonat, sowie gegebenenfalls
c) ein weiteres nicht- oder anionisches Tensid

in wäßriger, gegebenenfalls gepufferter Lösung enthält.

Das erfindungsgemäße Mittel enthält zweckmäßigerweise in wäßriger, gepufferter Lösung

a) 0,5 bis 15, bevorzugt 1,0 bis 12 Gew.-% polyethoxyliertes Triglycerid mit einem HLB-Wert von 4 bis 14,
b) 0,5 bis 10, bevorzugt 1,0 bis 7 Gew.-% sekundäres n-Alkansulfonat, sowie gegebenenfalls
c) 0,2 bis 5, bevorzugt 0,5 bis 3 Gew.-% eines weiteren nicht- oder anionischen Tensids.

Besonders bevorzugt ist ein Mittel folgender Zusammensetzung:

a) 2 bis 9 Gew.-% polyethoxyliertes Triolein oder Rizinusöl,
b) 1,2 bis 4,8 Gew.-% eines sekundären n-Alkansulfonats mit 12 bis 19 C-Atomen im Molekül oder eines Gemisches verschiedener sekundärer n-Alkansulfonate mit überwiegend 12 bis 19 C-Atomen im Molekül, sowie gegebenenfalls
c) 1 bis 2 Gew.-% eines gerad- oder verzweigtkettigen Alkylalkohol- oder Alkylarylalkohol-polyglykolethers mit durchschnittlich 3 bis 7 Oxyethyleneinheiten pro Molekül, eines Alkyl-arylsulfonates, sekundären Alkylsulfates oder eines Gemisches aus Alkansulfonaten mit 13 bis 15 C-Atomen im Molekül und 40 bis 50 % sekundärem Anteil

in wäßriger, gegebenenfalls gepufferter Lösung.

Als Puffer kann das erfindungsgemäße Mittel allgemein bekannte Puffersubstanzen enthalten, deren pK-Wert zwischen 2,0 und 10,0 liegt. Besonders bevorzugt sind Succinat-, Acetat-, Phosphat- oder Trispuffer. Die Puffer-Konzentration beträgt 5 bis 250, vorzugsweise 10 bis 170 mM.

Das erfindungsgemäße Mittel kann ferner zur Erhöhung der Ionenstärke ein Salz, beispielsweise Natriumchlorid, oder mehrere Salze enthalten. Die Salzkonzentration beträgt vorzugsweise 50 bis 200 mM, insbesondere 50 bis 150 mM.

Zusätzlich zu den genannten Komponenten kann das Reagens weitere Substanzen enthalten, die für die photometrische Analyse eines bestimmten Serumbestandteils erforderlich sind. Für den Fall von Spurenelementanalysen, wie z. B. Serum-Transferrin-Eisen, können diese ein Reduktionsmittel wie Ascorbinsaure sowie Farbkomplexbildner wie Bathophenanthrolin-Disulfonsaure, FerroZine® oder andere Verbindungen vom Ferroin-Typ darstellen.

Für die immunoturbidimetrische oder -nephelometrische Bestimmung von Serumbestandteilen, wie z. B. Apolipoproteinen und deren Untereinheiten sowie immunologisch aktiven Apolipoprotein-Spaltstücken, kann das Reagens als zusätzliche Komponenten Antikörper, z. B. in Form eines Antiserums, der daraus gewonnenen gamma-Globulin- oder IgG-Fraktion, oder auch monoklonale Antikörper sowie eine Substanz enthalten, die die Immunpräzipitationsreaktion fördert, wie Polyäthylenglykol mit einem Molekulargewicht von 1000 bis 10 000, vorzugsweise 6000, in einer Konzentration von 1 bis 6, bevorzugt 3 bis 4 Gew.-%.

Das erfindungsgemäße Verfahren und Reagens zur raschen und vollständigen Beseitigung von Trübungen in biologischen Flüssigkeiten ist hinsichtlich seiner aufhellenden Wirkung den bekannten Verfahren bzw. verfürbaren Mitteln deutlich überlegen, insbesondere bei Volumenverhältnissen von Serum : Reagens ⩾ 0,15. Die Überlegenheit zeigt sich besonders deutlich bei der Analyse von Spurenelementen im Serum, beispielsweise der Bestimmung von Eisen im Serum. Um eine ausreichende Meßgenauigkeit zu erzielen, müssen hierbei im Verhältnis zum Analysenreagens große Serummengen eingesetzt werden, d. h. in der Meßlösung ist mit einem hohen Lipidgehalt und damit verstärkter Trübungserscheinung zu rechnen. Durch die hohe Aufhellgeschwindigkeit lassen sich auch in stark lipämischen Seren Analysen bestimmter Bestandteile innerhalb von maximal 10 min nach Vermischen von Serum und Reagens durchführen, was besonders wichtig für die automatisierte Analyse ist.

Darüber hinaus ist es überraschend, daß sich trotz der im Vergleich zu dem nach DE-OS-2 829 531 beanspruchten Mittel hohen Detergenskonzentrationen in dem beschriebenen Reagens auch immunologische Bestimmungen von Serumbestandteilen über Antigen-Antikörper-Präzipitationsreaktionen einwandfrei durchführen lassen. Das erfindungsgemäße Verfahren und Reagens lassen sich daher auch in vorteilhafter Weise bei solchen Immunpräzipitationsreaktionen, beispielsweise bei der immunologischen Bestimmung von Serum-Apolipoproteinen bzw. deren Untereinheiten oder von immunologisch aktiven Apolipoprotein-Bruckstücken, einsetzen.

Die beigefügten Abbildungen stellen dar:

Abb. 1: Geschwindigkeit der Trübungsbeseitigung in einem stark lipämischen Serum mit verschiedenen Aufklarungsmitteln gemäß Beispiel 1.
A: Reagens A = Mittel gemäß EP-OS-0 041 704.
B: Reagens B = Erfindungsgemäßes Mittel.
C: Reagentienleerwert.
Volumenverhältnis Serum : Analysenreagens = 0,2; Temperatur = 25°C; λ = 578 nm, Schichtdicke = 1 cm.

Abb. 2: Immunoturbidimetrische Bestimmung von Serum-Apolipoprotein A-I (APO A-I): über Standard-Serum-Verdünnungsreihe ermittelte Eichkurve. Messung entspr. Beispiel 3.

Abb. 3: Immunoturbidimetrische Bestimmung von Serum-Apolipoprotein A-II (APO A-II); über Standard-Serum-Verdünnungsreihe ermittelte Eichkurve. Messung entspr. Beispiel 3.

Abb. 4: Immunoturbidimetrische Bestimmung von Serum-Apolipoprotein B (APO B); über Standard-Serum-Verdünnungsreihe ermittelte Eichkurve. Messung entspr. Beispiel 3.

Die folgenden Beispiele erläutern die Erfindung weiter:

**Beispiel 1**

Aufklarungsgeschwindigkeit eines lipämischen Serums mit verschiedenen, oberflächenaktive Mittel enthaltenden Reagentien

1. Reagentien

Reagens A (Mittel gemäß EP-OS-0 041 704)

| Inhaltsstoff | Konzentration |
| --- | --- |
| Mersolat® H, 30-%-ig in $H_2O$ | 150 ml/L |
| Triton® X 151, 30-%-ig in $H_2O$ | 200 ml/L |
| p-Toluolsulfonsäure, Na-Salz | 258 mmol/L |

Mersolar® H = eine im Handel erhältliche Mischung sekundärer Alkansulfonate mit 13 bis 14 C-Atomen und 40 bis 50 % sekundärem Anteil

Triton® X 151 = Isooctylphenol-polyethylenglykolether.

Reagens B (erfindungsgemäßes Mittel)

| Inhaltsstoff | Konzentration |
| --- | --- |
| Mulsifan® RT 163 | 90,00 8/L (=9,0 Gew.-%) |
| Hostapur® AT | 80,00 g/L (=4,8 Gew.-%) |
| Elfan® WA 50 | 40,00 g/L (=2,0 Gew.-%) |
| Natrium-Acetat-Puffer (pH 5,4) | 170 mmol/L |

2. Testdurchführung

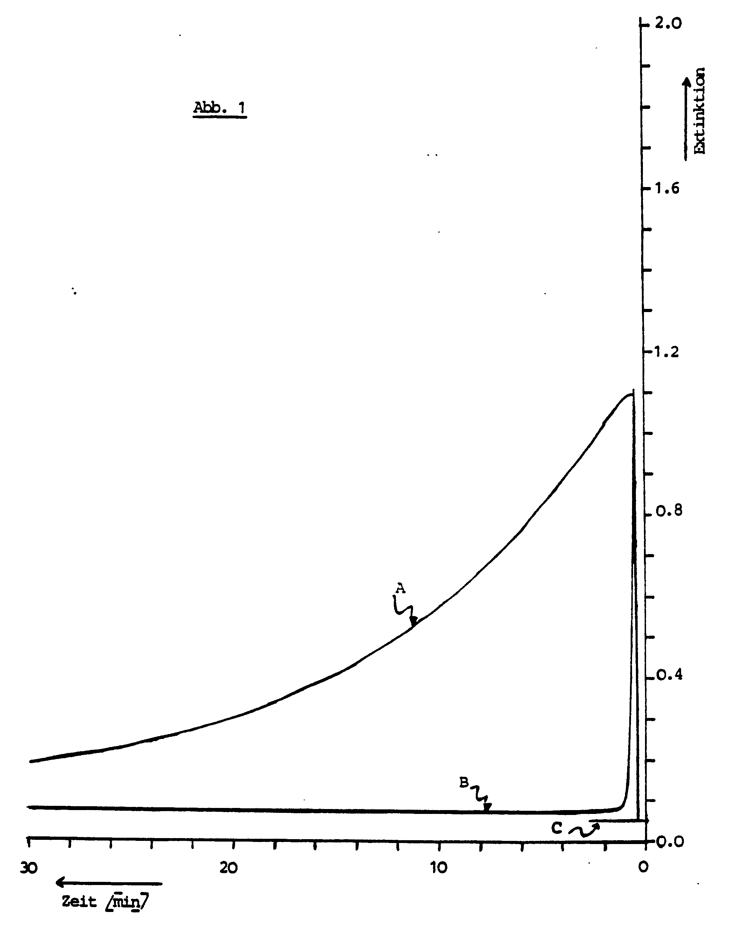

In einer 1 cm Küvette werden bei 25°C 0,2 ml eines stark lipämischen Serums mit 1 ml Reagens A bzw. Reagens B vermischt. Die Änderung der Durchlässigkeit bei 578 nm wird in Abhängigkeit von der Zeit bestimmt. Die erhaltenen Ergebnisse sind in Abb. 1 graphisch dargestellt.

3. Auswertung

Der in Abb. 1 dargestellte Verlauf der Änderung der Durchflässigkeit zeigt, daß bei einem Serum: Analysenreagens = 0,2 mit dem erfindungsgemäßen Mittel (Reagens B) bereits nach 2 min. eine vollständige Beseitigung der Trübung erreicht worden ist. Mit dem Reagens gemäß EP-OS-0 041 704 ist die Trübung auch nach 30 min. noch nicht vollständig beseitigt.

**Beispiel 2 (Anwendungsbeispiel):**

Bestimmung von Eisen im Serum

1. Reagentien

1.1 Leerwert-Reagens

| Inhaltsstoff | Konzentration |
| --- | --- |
| Mulsifan® RT 163 | 90,00 g/L (= 9,0 Gew.-%) |
| Hostaput® AT | 80,00 g/L (= 4,8 Gew.-%) |
| Elfan® WA 50 | 40,00 g/L (= 2,0 Gew.-%) |
| Natrium-Acetat-Puffer (pH 5,4) | 170 mmol/L |
| Ascorbinsäure | 10 mmol/l |

1.2 Farbreagens

| Inhaltsstoff | Konzentration |
| --- | --- |
| Mulsifan® RT 163 | 90,00 g/L (= 9,0 Gew.-%) |
| Hostapur® AT | 80,00 g/L (= 4,8 Gew.-%) |
| Elfan® WA 50 | 40,00 g/L (= 2,0 Gew.-%) |
| Natrium-Acetat-Puffer (pH 5,4) | 170 mmol/L |
| Ascorbinsäure | 10 mmol/L |
| FerroZine® [1] | 1,6 mmol/L |

[1] Eingetragenes Warenzeichen der Hach Chemical Co., Ames, Iowa, USA.

2. Testansatz

Temperatur: 37°C; Wellenlänge: 578 nm; Schichtdicke: 10 mm.
In eisenfreie Reaktionsgefäße werden folgende Lösungen pipettiert:

| | Probe | Probenleerwert |
|---|---|---|
| Farbreagens | 1,00 ml | - |
| Leerwert-Reagens | - | 1,00 ml |
| Serum | 0,20 ml | 0,20 ml |

Es wird jeweils vermischt, 10 min. inkubiert und danach Absorption von Probe gegen eine Mischung aus 1,00 ml Farbreagens und 0,20 ml $H_2O$ ($\Delta A_1$) sowie von Probenleerwert gegen eine Mischung aus 1,00 ml Leerwert-Reagens und 0,20 ml $H_2O$ ($\Delta A_2$) gemessen. Daraus wird $\Delta A = \Delta A_1 - \Delta A_2$ berechnet.

3. Auswertung

Die Konzentration von Eisen im Serum wird nach folgender Gleichung
Konzentration von Eisen im Serum (µg/100 ml) = Δ A x 1330
berechnet.

Anstelle von Mulsifan® RT 163 kann in den Resgentien 1.1 und 1.2 Mulsifan® RT 7 in gleichen Mengen eingesetzt werden, ohne daß sich das Ergebnis ändert. Desgleichen kann Elfan® WA 50 (Na-Dodecylbenzolsulfonat, 50 % Wirkstoffanteil in wäßriger Lösung) durch eine gleiche Konzentration (bezogen auf den Wirkstoffanteil) sn sekundärem Alkylsulfat (Teepol® 610 S, Fa. Shell) (8 bis 18 C-Atome im Molekül) ersetzt werden. In allen Fällen ist bei Verwendung lipämischen Serums die Aufklarung innerhalb der Inkubationsperiode abgeschlossen und vollständig.

**Beispiel 3 (Anwendungsbelspiel)**

Bestimmung von Apo-Lipoproteinen (A - I, A - II und B) im Serum

1. Reagentien 1.1 Schaf-anti-human Apo-A-I-Antiscrum (gamma-Globulinfraktion, Boehringer Mannheim GmbH, Kat.-Nr. 726 478) 1.2 Schaf-anti-human Apo-A-II-Antiserum (gamma-Globulinfraktion, Boehringer Mannheim GmbH, Kat.-Nr. 726 486) 1.3 Schaf-anti-human Apo-B-Antiserum (gamma-Globulinfraktion, Boehringer Mannheim GmbH, Kat .Nr 726 494)

1.4 Antiserum-Diluens Zusammensetzung

| Inhaltsstoff | Konzentration |
|---|---|
| Mulsifan® RT 163 | 20 g/L (= 2,0 Gew.-%) |
| Hostapur® AT | 20 g/L (= 1,2 Gew.-%) |
| Lutensol® ON 50 | 10 g/L (= 1,0 Gew.-%) |
| Kalium-Phosphat-Puffer (pH 7,4) | 10 mmol/L |
| NaCl | 150 mmol/L |
| Polyethylenglykol 6000 | 40 g/L |

1.5 Proben-Diluens (für Serum oder Standard); Kalium-Phosphat-Puffer (pH 7,4) 10 mmol/L, NaCl 150 mmol/L.

1.6 Standard-Serum (Immunoneph® Reference Standard, Immuno GmbH, Kat.-Nr. 4380105)

2. Antiserum-, Standard-Serum- und Serumverdünnung

2.1 Antiserum-Verdünnung

Antiserum (1.1, 1.2 oder 1.3) jeweils 10-fach mit Serum-Diluens 1.4 verdünnen. Vor Gebrauch 15 min bei 20 - 25° C stehen lassen.

2.2 Standard-Serum-Verdünnung

Standard-Serum (1.6) 5-, 10-, 20-, 40- und 80-fach mit Proben-Diluens (1.5) verdünnen.

2.3 Serum-Verdünnung

Für Apo A-I-Bestimmung Serum 20-fach, für Apo A-II- oder Apo B-Bestimmung Serum 10-fach mit Proben-Diluens (1.5) verdünnen.

3. Testansatz (für entweder Apo A-I, Apo A-II oder Apo-B) Temperatur: 25° C; Wellenlänge: 366 nm; Schichtdicke: 10 mm.

In Reaktionsgefäße wird pipettiert:

| | Probe | Probenleerwert |
|---|---|---|
| Antiserum-Verdünnung | 2,00 ml | - |
| Antiserum-Diluens | - | 2,00 ml |
| Serum- oder Standard-Serum-Verdünnung | 0,10 ml | 0,10 ml |

Es wird vermischt, 2,S Stunden inkubiert, kurz aufgeschüttelt und Absorption der Probe gegen eine Mischung aus 2,00 ml Antiserum-Verdünnung und 0,10 ml Probendiluens ($\Delta A_1$) sowie des Probenleerwerts gegen eine Mischung aus 2,00 ml Antiserum-Diluens und 0,10 ml Serum- oder Standard-Verdünnung ($\Delta A_1$) gemessen. Daraus wird $\Delta A = \Delta A_1 - \Delta A_2$ berechnet.

4. Auswertung

Die Konzentration von entweder Apo A-I, Apo A-II oder Apo B mit $\Delta A$ wird über die mit der Standardverdünnungsreihe aufgestellten Bezugskurven ermittelt.

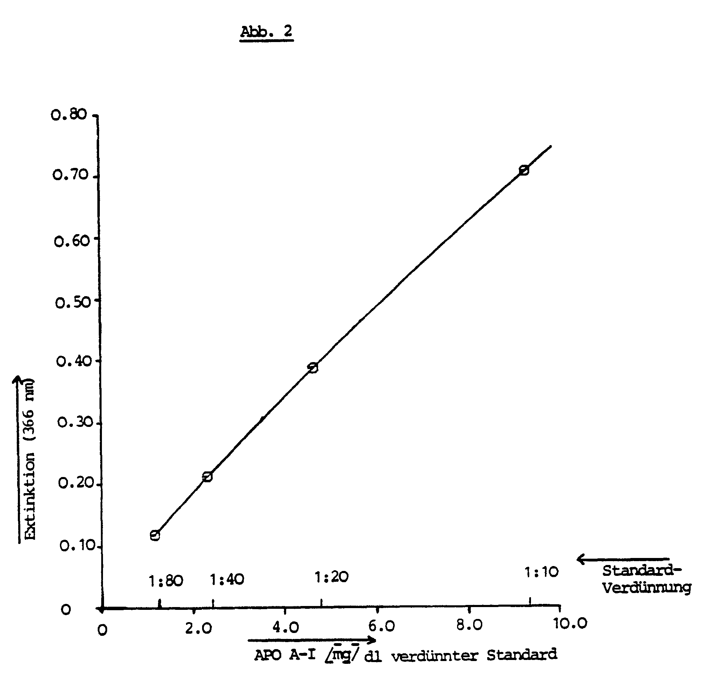

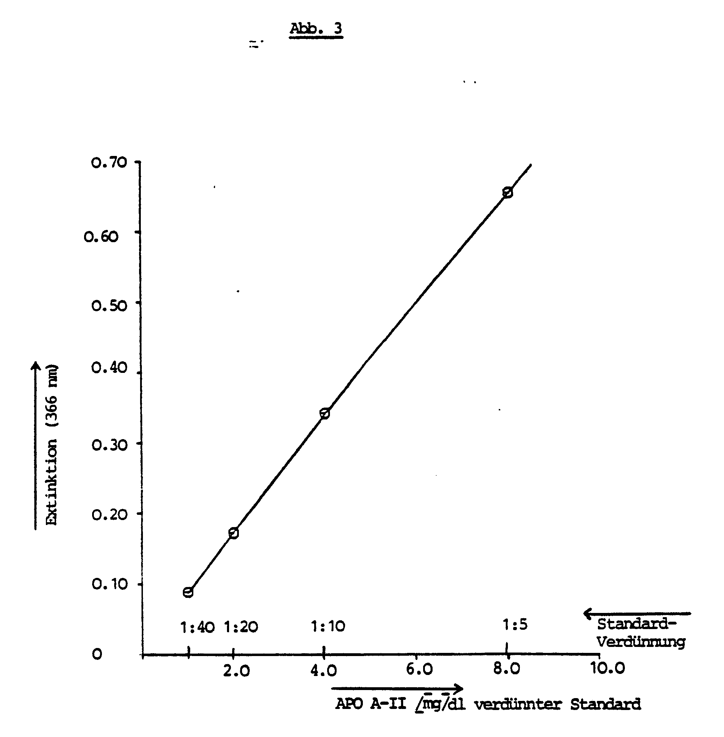

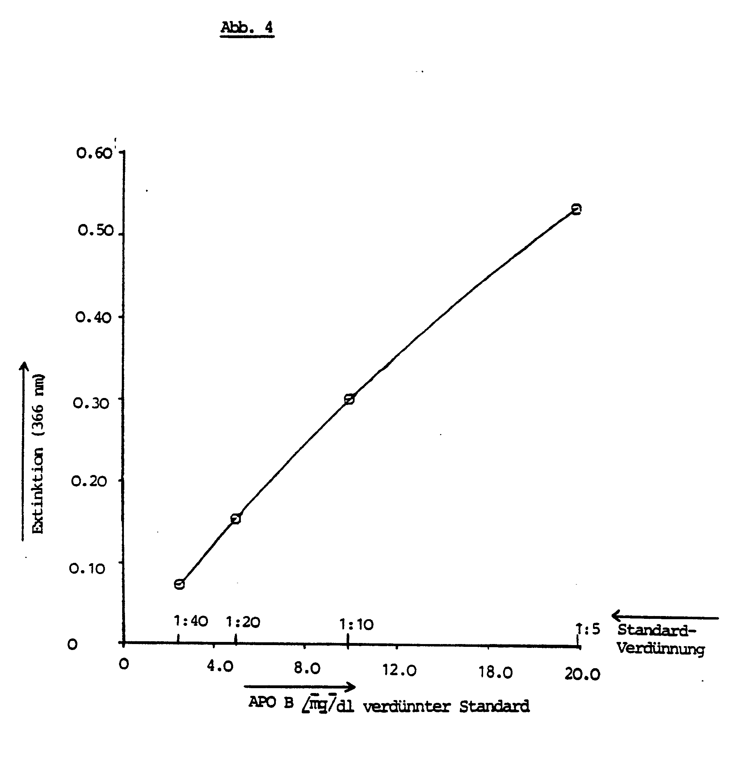

Typische, mit diesem Testverfahren erhaltene Bezugskurven sind in den Abb. 2 bis 4 dargestellt.

Anstelle von Mulsifan® RT 163 können im Antiserum-Diluens 1.4 auch Mulsifan® RT 7 oder polyethoxyliertes Triolein (z. B. Tagat® TO) in gleichen Mengen eingesetzt werden, ohne daß sich die Resultate ändern. Die Aufklarung lipämischer Seren ist in allen Fällen in kurzer Zeit (ca. 1 min) vollständig abgeschlossen, wie über den Probenleerwert-Ansatz ermittelt werden kann.

**Patentansprüche**

1. Verfahren zur Beseitigung von Trübungen in biologischen Flüssigkeiten durch Zusatz von oberflächenaktiven Mitteln, dadurch gekennzeichnet, daß als oberflächenaktive Mittel

a) ein polyethoxyliertes Triglycerid mit einem HLB-Wert von 4 bis 14,
b) ein sekundäres n-Alkansulfonat sowie gegebenenfalls
c) ein weiteres nicht- oder anionisches Tensid

in wäßriger, gegebenenfalls gepufferter Lösung eingesetzt werden.

2. Verfahren gemäß Anspruch 1, dadurch gekennzeichnet, daß ein Puffer mit einem pK-Wert zwischen 2,0 und 10,0 verwendet wird.

3. Verfahren gemäß Anspruch 2, dadurch gekennzeichnet, daß als Puffer ein Succinat-, Acetat-, Phosphat- oder Trispuffer in einer Konzentration von 5 bis 250 mM verwendet wird.

4. Verfahren gemäß einem der Ansprüche 1 bis 3, dadurch gekennzeichnet, daß zur Erhöhung der Ionenstärke wenigstens ein Salz zugesetzt wird.

5. Mittel zur Beseitigung einer Trübung in einer biologischen Flüssigkeit, dadurch gekennzeichnet, daß es

a) ein polyethoxyliertes Triglycerid mit einem HLB-Wert von 4 bis 14,
b) ein sekundäres n-Alkansulfonat sowie gegebenenfalls
c) ein weiteres nicht- oder anionisches Tensid

in wäßriger, gegebenenfalls gepufferter Lösung enthält.

6. Mittel gemäß Anspruch 5, dadurch gekennzeichnet, daß es einen Puffer mit einem pK-Wert zwischen 2,0 und 10,0 enthält.

7. Mittel gemäß Anspruch 6, dadurch gekennzeichnet, daß es als Puffer einen Succinat-, Acetat-, Phosphat- oder Trispuffer in einer Konzentration von 5 bis 250 mM enthält.

8. Mittel gemäß einem der Ansprüche 5 bis 7, dadurch gekennzeichnet, daß es zur Erhöhung der Ionenstärke wenigstens ein Salz enthält.

9. Verwendung eines Mittels gemäß einem der Ansprüche 5 bis 8 zur immunologischen Bestimmung von Serum-Apolipoproteinen.

10. Verwendung eines Mittels gemäß Anspruch 9, dadurch gekennzeichnet, daß die immunologische Bestimmung durch turbidimetrische oder nephelometrische Messung der Trübung erfolgt, die während der Immunpräzipitationsreaktion zwischen Apolipoprotein und dessen Antikörper entsteht.

**Claims**

1. Process for the removal of turbidities in biological fluids by addition of surface-active agents, characterised in that as surface-active agents there are used

a) a polyethoxylated triglyceride with an HLB value of 4 to 14,
b) a secondary n-alkane sulphonate, as well as possibly
c) a further non- or anionic tenside

in aqueous possibly buffered solution.

2. Process according to claim 1, characterised in that a buffer is used with a pH value between 2.0 and 10.0.

3. Process according to claim 2, characterised in that, as buffer, there is used on succinate, acetate, phosphate or tris buffer in concentration of 5 to 250 mM.

4. Process according to one of claims 1 to 3, characterised in that at least one salt is added for the increasing of the ionic strength.

5. Agent for the removal of a turbidity in a biological fluid, characterised in that it contains

a) a polyethoxylated triglyceride with an HLB value of 4 to 14,
b) a secondary n-alkane sulphonate, as well as possibly
c) a further non- or anionic tenside

in aqueous possibly buffered solution.

6. Agent according to claim 5, characterised in that it contains a buffer with a pK value between 2.0 and 10.0.

7. Agent according to claim 6, characterised in that, as buffer, it contains a succinate, acetate, phosphate or tris buffer in a concentraion of 5 to 250 mM.

8. Agent according to one of claims 5 to 7, characterised in that it contains at least one salt for the increasing of the ionic strength.

9. Use of an agent according to one of claims 5 to 8 for the immunological determination of serum apolipoproteins.

10. Use of an agent according to claim 9, characterised in that the immunological determination takes place by turbidimetric or nephelometric measurement of the turbidity which arises during the immune precipitation reaction between apolipoprotein and its antibody.

**Revendications**

1. Procédé pour l'éliminaiton de troubles dans les liquides biologiques, par addition d'agents tensioactifs, caractérisé en ce que, comme agent tensioactif, on utilise

a) un triglycéride polyéthocylé ayant un équilibre hydrophile-lipophile de 4 à 14,
b) un n-alcanesulfonate secondaire, ainsi qu'éventuellement,
c) un autre tensioactif non ionique ou anionique,

dans une solution aqueuse, éventuellement tamponnée.

2. Procédé selon la revendication 1, caractérisé en ce que l'on utilise un tampon ayant un pK compris entre 2,0 et 10,0.

3. Procédé selon la revendication 2, caractérisé en ce que comme tampon, on utilise un tampon succinate, acétate, phosphate ou tris, en une concentration de 5 à 250 mM.

4. Procédé selon l'une quelconque des revendications 1 à 3, caractérisé en ce que pour élever la force ionique, au ajoute au moins un sel.

5. Agent pour éliminer un trouble d'un liquide biologique, caractérisé en ce qu'il contient

a) un triglycéride polyéthoxylé ayant un équilibre hydrophile-lipophile de 4 à 14,
b) un n-alcanesulfonate secondaire, ainsi qu'éventuellement,
c) un autre tensioactif non ionique ou anoinique,

dans un solution aqueuse, éventuellement tamponnée.

6. Procédé selon la revendication 5, caractérisé en ce qu'il contient un tampon ayant un pK compris entre 2,0 et 10,0.

7. Procédé selon la revendication 6, caractérisé en ce que comme tampon, on utilise un tampon succinate, acétate, phosphate ou tris, en une concentration de 5 à 250 mM.

8. Agent selon l'un des revendications 5 à 7, caractérisé en ce que pour élever la force ionique, il contient au moins un sel.

9. Application d'un agent selon l'une des revendications 5 à pour la déterminaiton immunologique d'apolipoprotéines du sérum.

10. Application d'un agent selon la revendication 9, caractérisé en ce que la déterminaiton immunologique s'effectue par mesure turbidimétrique ou néphélométrique du trouble, qui, se forme pendant la réaction d'immunoprécipitation entre l'apolipoprotéine et son anticorps.

Abb. 1
Extinktion
2.0
1.6
1.2
0.8
0.4
0.0
A
B
C
30
20
10
0
Zeit /min/

Abb. 2

0.80
0.70
0.60
0.50
0.40
0.30
0.20
0.10
0
Extinktion (366 nm)
1:80
1:40
1:20
1:10
Standard-
Verdünnung
0
2.0
4.0
6.0
8.0
10.0
APO A-I [mg] dl verdünnter Standard

Abb. 3

0.70
0.60
0.50
0.40
0.30
0.20
0.10
0
Extinktion (366 nm)
1:40 1:20
1:10
1:5
Standard-
Verdünnung
2.0
4.0
6.0
8.0
10.0
APO A-II /mg/dl verdünnter Standard

Abb. 4

0.60
0.50
0.40
0.30
0.20
0.10
0
Extinktion (366 nm)
1:40
1:20
1:10
1:5
Standard-
Verdünnung
0
4.0
8.0
12.0
18.0
20.0
APO B [mg]/dl verdünnter Standard